(19) **Europäisches Patentamt**
**European Patent Office**
**Office européen des brevets**

(11) **EP 1 531 975 B1**

(12) **EUROPEAN PATENT SPECIFICATION**

(45) Date of publication and mention
of the grant of the patent:
**04.04.2007 Bulletin 2007/14**

(51) Int Cl.:
***B26D 5/02*** *(2006.01)*   ***B26D 5/08*** *(2006.01)*
***B26D 7/26*** *(2006.01)*

(21) Application number: **03763077.9**

(22) Date of filing: **01.07.2003**

(86) International application number:
**PCT/US2003/020693**

(87) International publication number:
**WO 2004/004991 (15.01.2004 Gazette 2004/03)**

(54) **ROTARY APPARATUS FOR SEVERING WEB MATERIALS**

DREHVORRICHTUNG ZUM DURCHTRENNEN VON BAHNMATERIALIEN

APPAREIL ROTATIF POUR LA COUPE DE MATERIAUX EN BANDE

(84) Designated Contracting States:
**AT BE BG CH CY CZ DE DK EE ES FI FR GB GR HU IE IT LI LU MC NL PT RO SE SI SK TR**

(30) Priority: **02.07.2002 US 187125**

(43) Date of publication of application:
**25.05.2005 Bulletin 2005/21**

(73) Proprietor: **THE PROCTER & GAMBLE COMPANY Cincinnati, Ohio 45202 (US)**

(72) Inventors:
 • **WELCH, David, Porter**
   **West Chester, OH 45069 (US)**
 • **VAN VALKENBURG, Curtis, H.**
   **Milford, OH 45150 (US)**

(74) Representative: **Borbach, Markus et al**
   **Procter & Gamble Service GmbH,**
   **Sulzbacher Strasse 40-50**
   **65824 Schwalbach am Taunus (DE)**

(56) References cited:
**EP-A- 0 312 703      US-A- 4 226 150**
**US-A- 4 240 312      US-B1- 6 173 633**

**Description**

**FIELD OF THE INVENTION**

This invention relates to apparatus for severing continuous webs or discrete sheets of materials, and more particularly for severing continuous webs or discrete sheets of materials having relatively high elongation and/or energy required at failure (e.g., polypropylene, NYLON, PET), which are often used in manufacturing of disposable absorbent articles.

**BACKGROUND OF THE INVENTION**

Rotary apparatus, such as rotary dies and flex blades, are well known in the art of severing web materials - including continuous webs or discrete sheets - that are generally used in production of disposable absorbent articles, as well as in other processes utilizing such materials. Rotary apparatus usually involve the use of oppositely rotating rolls, one of which may carry one or more severing tools - such as dies or flex blades -, and another roll may serve as an anvil against which the material is severed under a compression force by the tool.

US 4,226,150 discloses a die-cutting roll press and a means for adjusting the spaces between the axis of the anvil roll and the die roll of such a press. US 6,173,663 discloses a variable length rotary cutting system for cutting sheet material in various desired cut lengths. EP 312703 discloses a cutting apparatus comprising one cylinder having a cutting blade and one cylinder having an anvil surface, the apparatus being suitable to cut length from a web of low density material. It is disclosed that the clearance between the blade and the anvil surface can be adjusted by moving the cylinders axially relative to each other. The cylinders have each a pair of cylindrical bearers which are in contact and rolling engagement. These bearers are used to ensure a constant distance between the axes of the die cylinders. The "compression force" refers herein to a load applied substantially perpendicular to a web material disposed between a severing tool and an anvil during severing of the web material in a rotary apparatus.

The rotary apparatus, which employ one or more severing dies for cutting out various shapes from a web material, are generally known as rotary dies. The rotary dies are capable to cut in any direction- a machine direction MD, a cross machine direction CD, and/or any intermediate direction ID extending between the MD and the CD. Alternatively, the rotary apparatus which employ one or more flex blades are generally known as flex blade apparatus, and are capable of cutting generally in the cross-machine direction. (The term "machine direction" or "MD" refers herein to a direction of travel of the web material in a production process. The term "cross-machine direction" or "CD" refers to a direction that is generally perpendicular to the MD, and the term "intermediate direction" or "TD" refers to any direction extending between the MD and the CD.)

As the rolls rotate, when the tool and the anvil meet to sever the web material, the compression force applied between the tool and the anvil is an important factor affecting quality and efficiency of the operation. This is because the compression force affects the wear of the tool and, therefore, the longevity of the tool, affecting the frequency of downtime of the rotary apparatus required for changing or repositioning the tool, as well as cost of periodical regrinding and reinstalling of the apparatus itself.

The amount of compression force that the cutting tool exerts on the web material depends upon the engagement of the tool against the anvil surface. This is particularly important for materials that are generally known as "hard-to-cut materials." The term "hard-to-cut materials" refers herein to polymers with relatively high elongation and/or high energy required for failure, including such materials as polypropylene (PP), NYLON, polyethylene terephthalate (PET), and any combination thereof, provided in a film form or in a non-woven form. The hard-to-cut materials provided in a non-woven form are generally more difficult to cut than the same polymer in a film form.

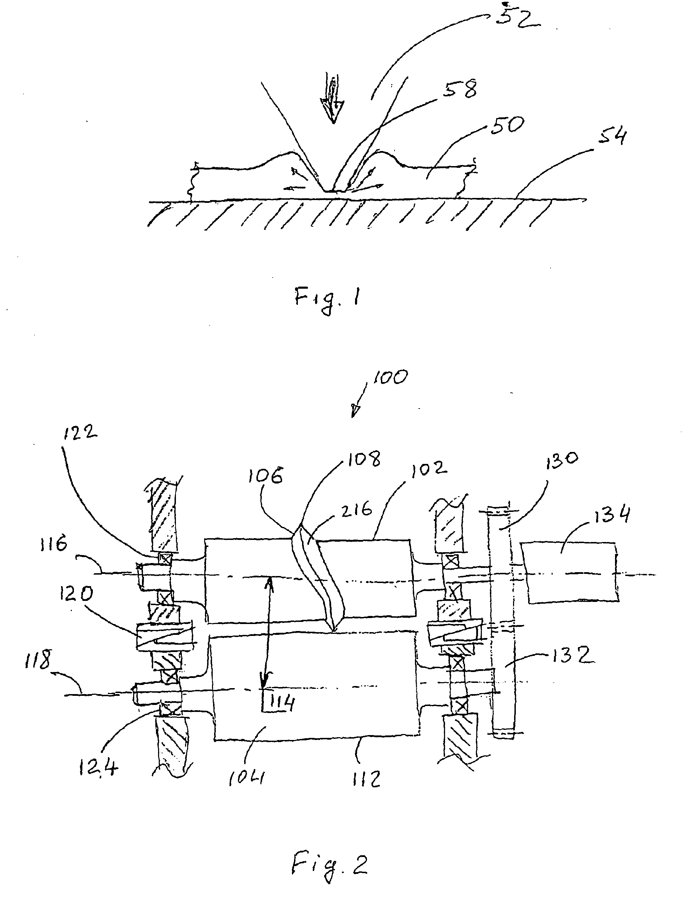

The above hard-to-cut materials generally do not "burst" under the compression force accompanied the severing operation (as often do the materials having relatively brittle failure characteristics), but as shown in Fig. 1, a hard-to-cut web material 50 is compressed and displaced between a severing tool 52 and an anvil surface 54, forming a thin membrane 56 under the severing edge 58 of the severing tool 52. The thin membrane 56 can be very difficult or even not possible to sever at very high compression forces. Therefore, the above hard-to-cut materials generally require relatively greater compression forces than the materials having relatively brittle failure characteristics, thus resulting in more rapid tool wear.

In addition, the hard-to-cut materials generally require greater accuracy in setting the severing tool in relation to the anvil surface than that normally required for materials having relatively brittle failure characteristics, and thus, capable to "burst" under compression. Even relatively small differences in the setting of the tool in relation to the anvil surface may result in substantial changes of the compression force, which, in turn, may affect the longevity of the tool. The accuracy of the engagement may become even more important for relatively large severing tools, when even a very small misalignment of the tool in relation to the anvil surface may subject a part of the tool to excessive forces, resulting in accelerated wear or rapid failure of that part of the tool.

Still further, during severing of the web material, as the severing tool wears and deteriorates, the quality of the severing may also deteriorate gradually or rapidly. Gradual deterioration of the severing tool usually takes place when the severing tools are made of tool steel. The severing edge of such a tool can deteriorate gradually by becoming duller, and, thus, result in gradual deterioration of the quality of the severing, but which often can be, at least temporarily, restored by reinstalling the tool engagement or by increasing the compression force. One way to increase the compression force is to move the severing tool radially toward the anvil. However, moving the tool during the severing operation often require a shutdown of the rotary apparatus, thus resulting in a downtime. Therefore, in order to extend the time between shutdowns, the severing tools made of tool steel are usually set initially to provide a greater compression force than that immediately needed. With respect to the instances of rapid failure of severing tools made of the so called "hard materials", such as carbides, ceramics, or cermets which usually last significantly longer than the severing tools made of tool steel and quality of the severing usually lasts longer also, the "hard-material" severing tools usually fail rapidly without warning resulting typically in the breakage of a portion of the "hard-metal" tool and the installation of a new "hard-materials" tool. Thus, in both examples above, the deterioration of the tool due to excessive compression forces, result in undesirable machine shutdowns and production downtimes.

Yet, another drawback of a conventional rotary apparatus is that the apparatus can require different engagement, which can include a gap or interference, between the tool and the anvil at lower rotational speeds than at higher rotational speeds, i.e., a greater compression force required between the tool and the anvil at lower rotational speeds than at higher rotational speeds. Therefore, often the tools are set up for engagements suitable for severing at lower rotational speeds to ensure satisfactory severing of the web material during machine startup. However, severing at higher rotational speeds (i.e., at production speeds after machine startup) with engagements suitable for lower rotational speeds can result in excessive compression forces between the tool and the anvil during the higher rotational speeds. Again, the effect can be accelerated wear of the tool at the higher production speeds.

Thus, conventional rotary apparatus exhibit a number of negative characteristics related to high compression forces between the severing tool and the anvil, resulting in various operational deficiencies.

Accordingly, it would be desirable to provide a rotary apparatus that overcomes the disadvantages exhibited by conventional rotary apparatus. Specifically, it would be desirable to provide a rotary apparatus that enables to sever the hard-to-cut materials at lower compression forces between the severing tool and the anvil.

Subsequently, it has been surprisingly discovered by the Applicants that when a speed ratio, i.e., a ratio between an anvil surface linear velocity and a severing edge of the severing tool linear velocity of the oppositely rotating severing tool and anvil surface, ranges from greater than about 1.02 to about 1.25, or from about 1.05 to about 1.10 (as was more often practiced by the Applicants), the operating efficiency of a severing operation can be substantially improved due to lower compression forces than that are normally required for severing web material , especially, the hard-to-cut web materials by conventional severing processes.

For example, Fig. 2 illustrates a rotary apparatus 100 developed and commercially utilized by the Applicants for severing the hard-to-cut materials. The rotary apparatus 100 comprises a tool roll 102 and an anvil roll 104. The tool roll 102 comprises a severing tool 106 having a severing edge 108. The anvil roll 110 comprises anvil surface 112, wherein the compression force is set by adjusting the distance 114 between the axes 116 and 118 of the respective tool rolls 102 and 104 via adjustable wedges 120 -- which can be alternatively any suitable mechanism capable of adjusting the distance 114, such as, for example, an eccentric and the like -- separating bearings 122 and 124 of the respective rolls 102 and 104. The rotary apparatus 100 employs a speed ratio SR between a linear velocity of the anvil surface VA and a linear velocity of the severing edge VS. The speed ratio can be provided, for example, by a desired gear ratio between gears 130 and 132, each one driving the respective rolls 102 and 104, from a suitable motor 134.

However, as was subsequently discovered by the Applicants, the rotary apparatus 100 is generally limited to employing the severing tool 106 made from tool steel materials, but not from so called "hard materials" such as carbides, ceramics, or cermets materials, due to high compression forces that can develop between the severing edge 108 and the anvil surface 112 due to possible instances of instability of the distance 114, which can change due to different thermo-expansions in the tool roll 102 and the anvil roll 104 in comparison to the bearings 122 and 124 and the adjusting wedges 120. The change in the distance 114 can affect the compression force between the severing edge 108 and the anvil surface 112. For example, if the distance 114 is reduced, the compression force is increased. Although, the severing tools made of the above-listed "hard materials" can withstand substantially greater compression forces than a severing tool made from tool steel, the instabilities in the distance 114 above can result in conditions at which these "hard materials" are more susceptible to rapid failure.

Therefore, it would be further desirable to provide a rotary apparatus that overcomes the disadvantages exhibited by conventional rotary apparatus and by the apparatus 100 above. Specifically, it would be desirable to provide a rotary apparatus that can sever the hard-to-cut materials at lower compression forces between the tool and the anvil, and is capable of employing severing tools made of carbides, ceramics, cermets, or tool steel materials.

## SUMMARY OF THE INVENTION

In response to the difficulties and problems discussed above, a new rotary apparatus capable of severing the hard-to-cut materials at lower compression forces between the tool and the anvil and capable of employing severing tools made of carbides, ceramics, cermets, or tool steel materials -- has been discovered.

The rotary apparatus of the present invention includes a tool roll capable of rotating around a tool roll axis and including at least one severing tool having a severing edge capable of rotating at a diameter D1 around the tool roll axis at a severing edge linear velocity VS, and at least one tool bearer ring capable of rotating around the tool roll axis at a diameter D3. The rotary apparatus further includes an anvil roll disposed substantially parallel and opposite the tool roll and capable of rotating around an anvil roll axis, and including an anvil surface capable of rotating at a diameter D2 around the anvil roll axis at an anvil linear velocity VA, and at least one anvil bearer ring disposed opposite the at least one tool bearer ring and capable of rotating around the anvil roll axis at a diameter D4 and disposed opposite the at least one tool bearer ring, the at least one tool bearer ring and the at least one anvil bearer ring being in contact with each other suitable to counter-rotate the tool roll and the anvil roll in relation to each other,

wherein D1+ D2 = D3 + D4, and

wherein a speed ratio SR is equal

$$SR = \frac{VA}{VS} = \frac{D3}{D1} \times \frac{D2}{D4}$$

and ranging from greater than about 1.02 to about 1.25.

In the present invention, the speed ratio SR can also range from about 1.05 to about 1.10. In addition, the severing tool can be a flex blade or a die having the severing edge configured to include severing edge portions selected from a group comprising, a rectilinear portion, a curvilinear portion, and any combination thereof. Further, the severing tool can be made from a material selected from the group consisting of tool steel, carbides, ceramics, cermets, and any combination thereof. Furthermore, the drive can be selected from a group consisting of a motor and a gear ratio communicating with the tool roll and the anvil roll, and two motors, a first motor communicating with the tool roll and a second motor communicating with the anvil roll.

## BRIEF DESCRIPTION OF THE DRAWINGS

While the specification concludes with claims particularly pointing out and distinctly claiming the subject matter that is regarded as the present invention, it is believed that the invention will be better understood from the following figures taken in conjunction with accompanying description in which like parts are given the same reference numeral:

Fig. 1 is a simplified, enlarged, side elevation view of a severing operation of a hard-to-cut web material, compressed and leaving a thin membrane between a severing tool and an anvil surface;

Fig. 2 is a simplified front elevation view of a rotary apparatus employing a speed ratio between an anvil surface linear velocity and a severing edge linear velocity, and which is generally limited to utilizing severing tools made only from tool steel materials;

Fig. 3 is a simplified front elevation view of one embodiment of a rotary apparatus of the present invention comprising a rotary die and embodying the essential features of the present invention;

Fig. 4 is a simplified side elevation cross-section view taken along line 4-4 of the rotary die of Fig. 3;

Fig. 5 is a rollout view of one embodiment of the severing edge of the rotary die of Fig. 3 and 4, forming an open-edge configuration;

Fig. 6 is a rollout view of another embodiment of the severing edge of the rotary die of Fig. 3 and 4, forming a closed-edge configuration;

Fig. 7 is a rollout view of yet another embodiment of the severing edge of the rotary die of Fig. 3 and 4, forming a closed-edge configuration;

Fig. 8 is a rollout view of yet another embodiment of the severing edge of the rotary die of Fig. 3 and 4, forming two closed-edge configurations comprising a common edge;

Fig. 9 is a simplified front elevation view of another embodiment of a rotary apparatus of the present invention having a cantilever design;

Fig. 10 is a simplified front elevation view of a rotary apparatus which does not form part of the present invention having a bearer ring, which is rotatably associated with a tool roll by a bearing, enabling independent rotation of the tool roll and the bearer ring;

Fig. 11 is a simplified front elevation view of another embodiment of a rotary apparatus of the present invention comprising a flex blade and embodying the essential features of the present invention;

Fig. 12 is a simplified side elevation cross-sectional view along line 12-12 of the flex blade apparatus of Fig. 11;

Fig. 13 is a simplified front elevation view of a rotary apparatus which does not form part of the present invention comprising a flex blade and embodying the essential features of the present invention; and

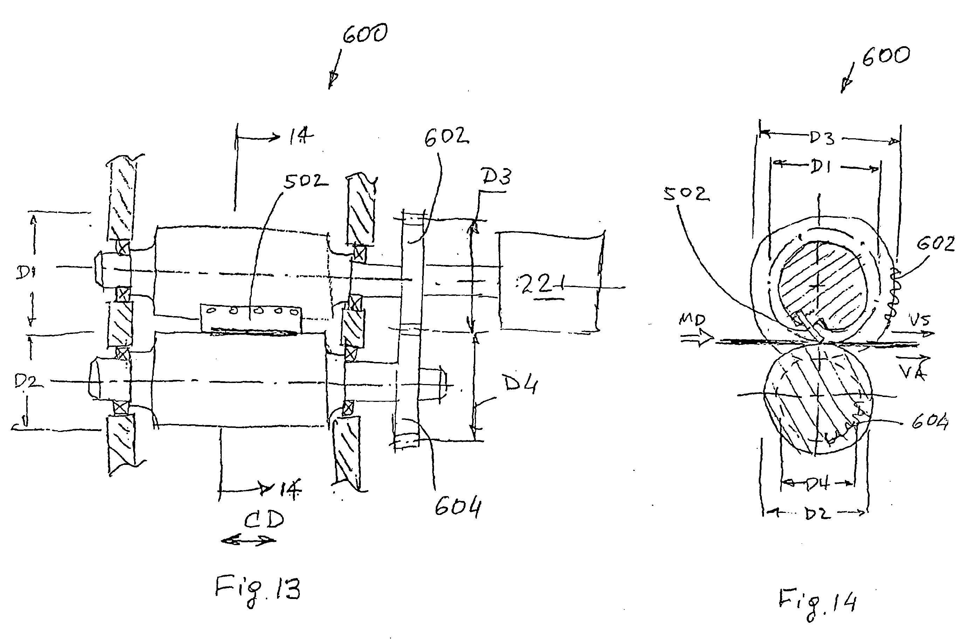

Fig. 1 is a simplified side elevation cross-sectional view along line 14-14 of the flex blade apparatus of Fig. 13.

## DETAILED DESCRIPTION OF THE INVENTION

The apparatus of the present invention can be used to sever continuous webs or discrete sheets of materials at substantially reduced compression forces than that normally taking place in conventional processes. The present invention can be particularly useful for severing the hard-to-cut materials having substantially high elongation and/or requiring a substantially high energy at failure, including such materials as polypropylene (PP), NYLON, polyethylene terephthalate (PET), and the like, in both film and non-woven forms.

The present invention can be especially useful for severing the above materials in a non-woven form, which is generally more difficult to sever than the materials in a film form, both of which are commonly used in manufacturing of disposable absorbent articles, as well as in many other productions. However, it should be noted that the Applicants believe that the present invention can be useful for severing any web material which has sufficient structural integrity to be processed as a continuous web or a discreet sheet, such as plastic films, non-woven substrates, metal foils, foams, rubbers, and other materials, either separately or in combination, in a single or multiple-layer forms.

However, for the purpose of simplicity, the present invention will be described in terms of preferred embodiments as shown in the drawings. Further, the present invention can be used with both types of severing tools: the dies and the flex blades.

It has been surprisingly discovered by the Applicants that when a "speed ratio" SR, which refers hereinafter to a ratio between an anvil surface linear velocity and a severing edge of the severing tool linear velocity of the oppositely rotating severing tool and anvil surface, ranges from greater than about 1.02 to about 1.25, or from about 1.05 to about 1.10 (as has been more often practiced by the Applicants), the operating efficiency of a severing operation can be substantially improved because of increased longevity of the severing tool affected by lower compression forces than that are normally required for severing, especially, the hard-to-cut materials by conventional severing processes.

Severing of web materials between a severing tool and an anvil surface, counter-rotating in relation to each other at respective linear speeds that differ from each other within about 2%, is known in the art. Such conditions often can take place when a speed ratio of about 1.02 or less is created initially between the anvil and the tool in order to compensate for future one or more regrinding of the tool between the periods of tool deterioration. Consequently, the subsequent regrinding(s) reduces or eliminates that initial speed ratio. The initial speed ratio of about 1.02 or less normally does not affect beneficially the longevity of the tool. In fact, it has been observed by the Applicants that the initial speed ratio of greater than about 1.00 to about 1.02 can negatively affect the longevity of the tool, resulting in premature deterioration or complete failure of the tool working under such conditions.

However, the Applicants discovered that when a speed ratio ranges from greater than about 1.02 to about 1.25, or from about 1.05 and about 1.10 (as has been more often practiced by the Applicants), or alternatively, from less than about 0.98 to about 0.80, or from about 0.95 to about 0.91 (as also has been more often practiced by the Applicants), the operating efficiency of a severing operation can be substantially improved due to lower compression forces than that are normally required for severing, especially, the hard-to-cut materials by conventional processes. This increase or decrease in the speed ratio provides a sufficient relative motion between the severing edge and the anvil surface, which facilitates the cutting and separation of the severed portion from the web.

Generally, a speed ratio of about 1.05 or about 0.95 are generally sufficient, however, lower or greater speed ratios specified above can be utilized as well, if desired.

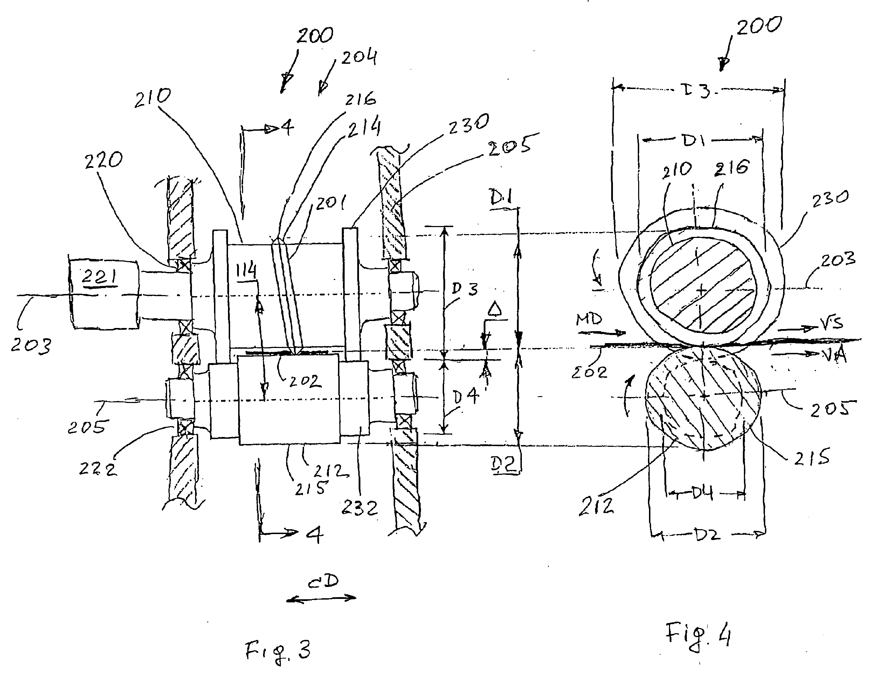

Fig. 3 and 4 illustrate one embodiment of a rotary apparatus 200 of the present invention, wherein a rotary die 201 is used to sever a web material 202. Fig. 3 shows a simplified front elevation view, and Fig. 4 shows a simplified side elevation cross-sectional view taken along line 4-4 of the apparatus 200 of Fig. 3. The rotary apparatus 200 comprises a pair of generally parallel, counter-rotating rolls 204, both of which can be rotatably mounted on a suitable frame 205.

The rotary apparatus 200 may be positioned vertically, horizontally, inclined, or in any other configuration. One of the rolls includes a tool roll 210 and the other roll includes an anvil roll 212, counter-rotating in opposite directions from each other. For example, if the tool roll 210 rotates in a counterclockwise direction, then the anvil roll 212 rotates in a clockwise direction and vice versa. The tool roll 210 and the anvil roll 212 can be rotatably supported within the frame 205 by any means including, for example, suitable bearings 220 and 222, supporting the tool roll 210 and the anvil roll 212, respectively.

The tool roll 210 can be a circular roll or any other shape roll, or any other mechanism or device which can be adapted to comprise one or more severing tool 214 (shown as a die 201) in a desired position to sever the web material 202 being fed between the tool roll 210 and the anvil roll 212. The die 201 can be monolithic with the tool roll 210 or can be attached thereto by any conventional means.

The die 201 can have a severing edge 216 configured to produce a cut in the web material 202 of any desirable configuration. The severing edge 216 can be configured to include severing edge portions selected from a group consisting of a rectilinear portion, a curvilinear portion, and any combination thereof, extending in any direction, such as the machine direction MD, the cross-machine direction CD, and/or any intermediate direction ID extending between the MD and the CD.

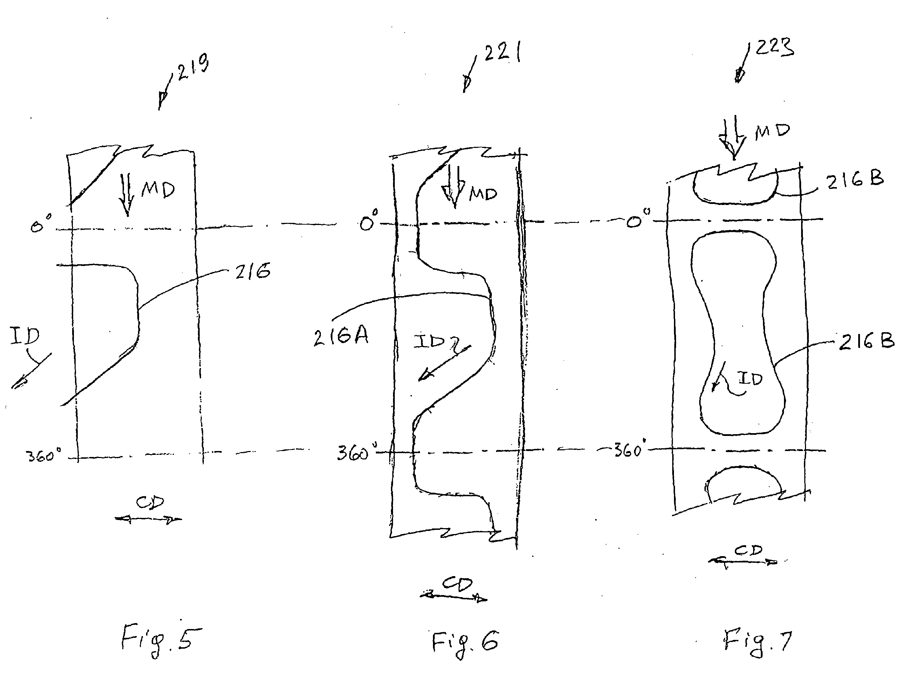

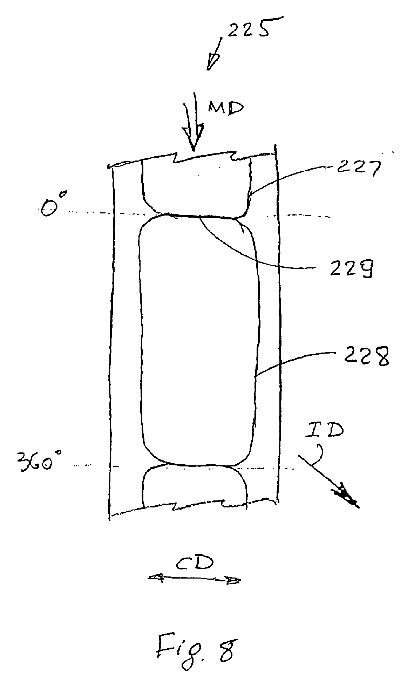

For example, Fig. 5 illustrates a rollout view of one embodiment of the severing edge 216 configured to make a curved cut defining an open-edge configuration 219, wherein various portions of the severing edge 216 make cuts in various directions: the MD, the CD, and the ID. Fig. 6 and 7 illustrate rollout views of other embodiments of closed-edge configurations of severing edges 216A and 216B, respectively, defining closed-edge configurations 221 and 223, respectively. Fig. 8 further illustrates a rollout view of yet another embodiment of a closed-edge configuration 225 comprising two closed-edge configurations 227 and 228, having a common severing edge 229.

Referring again to Fig. 3 and 4, the severing edge 216 of the die 201 rotates at a diameter D1 around an axis 203 of the tool roll 210 against an anvil roll 212 having an anvil surface 215 counter-rotating at a diameter D2 around an axis 205 of the anvil roll 212. Either one of the rolls 210 or 212 can be driven by any suitable driving means, attached directly or indirectly thereto, for example, by any suitable motor 221. Fig. 3 shows a motor 224 driving the tool roll 210, however, alternatively, the motor 224 can drive the anvil roll 212.

The rotary apparatus 200 also includes tool bearer rings 230, disposed on the tool roll 210, and anvil bearer rings 232 disposed on the anvil roll 212 and opposing the tool bearer rings 230. The tool bearer rings 230 and the anvil bearer ring 232 contact each other to form a sufficient frictional relationship between the tool bearer rings 230 and the anvil bearer rings 232 in order to drive the anvil roll 212 directly by the tool roll 210 driven by the motor 224. (Alternatively, if the anvil roll 212 is driven by the motor 224, then the above bearer rings will drive the tool roll 210.)

The bearer rings 230 and 232 can be monolithic with the respective rolls 210 and 212 or can be attached to the respective rolls 210 and 212 by any conventional means so the angular velocity of the tool bearer rings 230 is equal to the angular velocity of the tool roll 210, and the angular velocity of the anvil bearer rings 232 is equal to the angular velocity of the anvil roll 212. The bearer rings 230 and 232 provide a more consistent engagement between the severing edge 216 and the anvil surface 215 than that can be provided by the rotary apparatus 100 of Fig. 2, due to a substantially increased stability of the distance 114 between the axes 203 and 205 of the respective rolls 210 and 212, resulting from separating the possible negative affects of different thermo-expansions in the tool roll 210 and the anvil roll 212 in comparison to that in the bearings 220 and 222..

It should be understood that the number of bearer rings 230 and 232 could vary. For example, Fig. 9 shows a simplified front elevation view of another embodiment of a rotary apparatus 300 of the present invention having a cantilever design in relation to a frame 314 and comprising a tool roll 310 and an anvil roll 312. The rotary apparatus 300 includes at least one tool bearer ring 230 and at least one anvil bearer ring 232, corresponding with the tool bearer ring 230. With the cantilever design of the rotary apparatus 300, the number of bearer rings can be reduced to a single pair of bearer rings 230 and 232. Further, similarly to the rotary apparatus 200 of Fig. 3 and 4, either one of the rolls 310 or 312 of the rotary apparatus 300 can be driven by any suitable driving means, attached directly or indirectly thereto, for example, by any suitable motor 221. Fig. 9 shows a motor 221 driving the tool roll 310, however, alternatively, the motor 221 can drive the anvil roll 312.

Referring to Fig. 3, 4, and 9, the tool bearer rings 230 have a diameter D3, which is preferably greater than the diameter D1 of the rotation of the severing edge 216 by a difference Δ, in order to provide protection to the severing edge 216 from a possible accidental physical damage during handling of the tool roll. The anvil bearer rings 232 have a diameter D4, which in the shown embodiments 200 and 300, is smaller, by the same difference Δ, than the diameter D2 of rotation of the anvil surface 215. (Although, alternatively, the diameter D4 can be greater than the diameter D2 in the instances, as noted above, when D3 is smaller than D1.)

In order to maintain a desired relative position between the severing edge 216 and the anvil surface 215, providing a desired engagement between the severing edge 216 and the anvil surface 215, the relationship between the above diameters should satisfy the following equation:

$$D1 + D2 = D3 + D4 \qquad\qquad (1)$$

Then, the speed ratio SR, which again refers herein to a ratio between the anvil surface linear velocity VA of the anvil surface 215 and the severing edge linear velocity VS of the severing edge 216, can be determined by the following equation:

$$SR = \frac{VA}{VS} = \frac{D3}{D1} \times \frac{D2}{D4} \qquad\qquad (2)$$

As noted above, the severing edge 216 can be run under-speed or over-speed of the linear speed of the anvil surface 215. When the diameter D3 of the tool bearer rings 230 is greater than the diameter D1 of the severing edge 216 (as shown in Fig. 3, 4, and 9 in order to provide physical protection to the severing edge 216 during handling of the tool roll 210), the anvil surface linear velocity VA (see Fig. 4) of the anvil surface 215 is greater than the severing edge linear velocity VS (see Fig. 4) of the severing edge 216, wherein the speed ratio SR can be range from greater than about 1.02 to about 1.25, or from about 1.05 to about 1.10 (as more often practiced by the Applicants).

However, alternatively, when the diameter D3 of the tool bearer rings 230 is smaller than the diameter D1 of the severing edge 216, the anvil surface linear velocity VA of the anvil surface 215 is smaller than the severing edge linear velocity VS of the severing edge 216, wherein the speed ratio SR can range from less than about 0.98 to about 0.80, or from about 0.95 to about 0.91. This alternative does not form part of the invention.

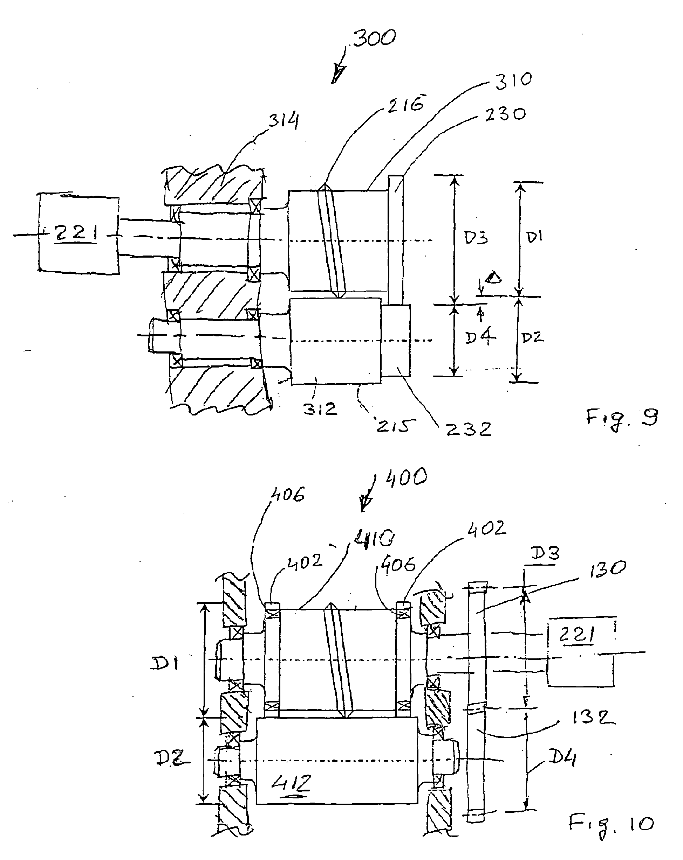

Fig 10 shows a simplified front elevation view of a rotary apparatus 400 which does not form part of the present invention having bearer rings 402 that are rotatably associated with a tool roll 410 by a bearing 406, enabling independent rotation of the tool roll 410 and the bearer ring 402. The speed ratio SR between the anvil surface linear velocity VA and the severing tool linear velocity VS can be adjusted by a drive that can be communicated with the tool roll 410 and the anvil roll 412. The drive can be any drive capable of providing independent rotations to the tool roll 410 and the anvil roll 412. For example, the drive can include a motor 221 and a desired gear ratio provided by gears 130 to 132 to result in the desired speed ratio SR above. Alternatively, the drive can include two motors, each one communicating with the tool roll 410 or the anvil roll 412 to provide the desired speed ratio SR. Further, alternatively, the bearer ring 402 can be associated not with the tool roll 410, but with the anvil roll 412, wherein the bearer ring 402 is in contact with the tool roll 410.

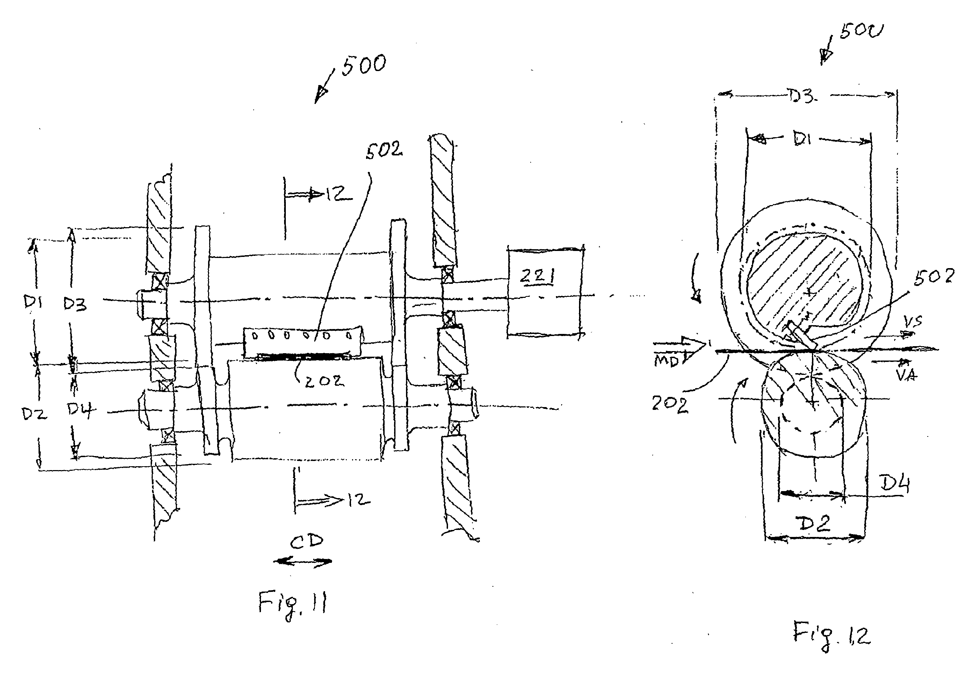

As noted above, the present invention can be used with one or more rotary dies 201 as shown in Fig. 3, 4, and 9 or with one or more flex blades 502 as shown in Fig. 11 and 12, illustrating a rotary apparatus 500. Fig. 11 shows a simplified front elevation view of the rotary apparatus 500, and Fig. 12 shows a simplified side elevation cross-sectional view taken along line 12-12 of the rotary apparatus 500 of Fig. 11. The flex blade 502 represents herein any conventional flex blade associated with a tool roll 504 in any conventional way. For example, the flex blade 502 of Fig. 11 and 12 represents one embodiment of a flex blade attached to the tool roll 504 using a fixed, cantilever beam design. Other conventional embodiments of the flex blade can include any conventional spring-loaded, air-loaded, or hydraulic-loaded flex blades, including sliding or bending configurations, or any other combination thereof.

The makeup of the rotary apparatus 500 having a flex blade 502 can be similar in all or any aspects to the rotary apparatus 200, 300, and 400 described in detail above. The relationship between the diameters D1, D2, D3, and D4 expressed in the equation (1) above, and the relationship between the speed ratio SR and the above diameters expressed and the equation (2) above can also be similar in all or any aspects to the rotary apparatus 200 and 300 above.

Fig. 13 shows another rotary apparatus 600 which does not form part of the present invention utilizing a flex blade 502, wherein the speed ratio SR can be provided by a desired gear ratio between a tool roll gear 602 and an anvil gear 604 engaged with each other and driven by any suitable motor 221 described above. The equations (1) and (2) above can be similarly applied for calculating the speed ratio SR by using the pitch diameter of the tool gear 602 as D3 and the pitch diameter of the anvil gear 604 as D4. The makeup of the rotary apparatus 600 having a flex blade 502 can be similar in all or any aspects to the rotary apparatus 200, 300, and 400 described in detail above. The relationship between the diameters D1, D2, D3, and D4 expressed in the equation (1) above, and the relationship between the speed ratio SR and the above diameters expressed and the equation (2) above can also be similar in all or any aspects to the rotary apparatus 200 and 300 above.

While particular embodiments and or individual features of the present invention have been illustrated and described, it would be obvious to those skilled in the art that various other changes and modifications can be made without departing from the spirit of the invention. Further, it should be apparent that all combinations of such embodiments and features are possible and can result in preferred executions of the invention.

**Claims**

1.   A rotary apparatus (200) suitable for severing a web material (202), the apparatus **characterized by** comprising:

   (a) a tool roll (210) capable of rotating around a tool roll axis, the tool roll (210) **characterized by** comprising :

      (i) at least one severing tool (214) having a severing edge capable of rotating at a diameter D1 around the tool roll axis at a severing edge linear velocity VS;
      (ii) at least one tool bearer ring capable of rotating around the tool roll axis at a diameter D3;

   (b) an anvil roll (212) disposed substantially parallel and opposite the tool roll 210 and capable of rotating around an anvil roll axis, the anvil roll **characterized by** comprising:

      (i) an anvil surface capable of rotating at a diameter D2 around the anvil roll axis at an anvil linear velocity VA;
      (ii) at least one anvil bearer ring (232) disposed opposite the at least one tool bearer ring (230) and capable of rotating around the anvil roll axis at a (230) diameter D4 and disposed opposite the at least one tool bearer ring (230), the at least one tool bearer ring 230 and the at least one anvil bearer ring (232) being in contact with each other suitable to counter-rotate the tool roll (210) and the anvil roll (212) in relation to each other,

   wherein D1 + D2 = D3 + D4, and
   wherein a speed ratio SR is equal

$$SR = \frac{VA}{VS} = \frac{D3}{D1} \times \frac{D2}{D4}$$

   and ranging from greater than 1.02 to 1.25.

2.   The rotary apparatus (200) of Claim 1, wherein the severing tool 214 is a flex blade or a die (201) having the severing edge configured to include severing edge portions selected from a group **characterized by** comprising, a rectilinear portion, a curvilinear portion, and any combination thereof.

3.   The rotary apparatus (200) of any one of the preceding claims, wherein the severing tool (214) is made from a material selected from the group consisting of tool steel, carbides, ceramics, cermets, and any combination thereof.

4.   The rotary apparatus (200) of any one of the preceding claims, wherein the drive is selected from a group consisting of a motor (224) and a gear ratio communicating with the tool roll and the anvil roll, and two motors, a first motor communicating with the tool roll and a second motor communicating with the anvil roll.

5.   The rotary apparatus (200) of any one of the preceding claims, wherein the bearer ring (230) is disposed on a bearing associated with the tool roll (210).

6.   The rotary apparatus (200) of any one of the preceding claims, wherein the bearer ring (230) is disposed on a bearing associated with the anvil roll (212).

**Patentansprüche**

1. Rotierende Vorrichtung (200), die dazu geeignet ist, ein Bahnenmaterial (202) zu trennen, wobei die Vorrichtung **dadurch gekennzeichnet ist, dass** sie Folgendes umfasst:

   (a) eine Werkzeugwalze (210), die in der Lage ist, sich um eine Werkzeugwalzenachse zu drehen, wobei die Werkzeugwalze (210) **dadurch gekennzeichnet, ist, dass** sie Folgendes umfasst:

   (i) mindestens ein Trennwerkzeug (214) mit einer Trennkante, die sich mit einem Durchmesser D1 mit einer linearen Trennkantengeschwindigkeit VS um die Werkzeugwalzenachse drehen kann;
   (ii) mindestens einen Werkzeugträgerring, der in der Lage ist, sich mit einem Durchmesser D3 um die Werkzeugwalzenachse zu drehen;

   (b) eine Ambosswalze (212), die im Wesentlichen parallel und gegenüber der Werkzeugwalze 210 angeordnet ist und in der Lage ist, sich um eine Ambosswalzenachse zu drehen, wobei die Ambosswalze **dadurch gekennzeichnet ist, dass** sie Folgendes umfasst :

   (i) eine Ambossoberfläche, die in der Lage ist, sich mit einem Durchmesser D2 mit einer linearen Ambossgeschwindigkeit VA um die Ambosswalzenachse zu drehen;
   (ii) mindestens einen Ambossträgerring (232), der gegenüber dem mindestens einen Werkzeugträgerring (230) angeordnet ist und in der Lage ist, sich um die Ambosswalzenachse mit einem Durchmesser D4 und gegenüber dem mindestens einen Werkzeugträgerring (230) angeordnet zu drehen, wobei der mindestens eine Werkzeugträgerring 230 und der mindestens eine Ambossträgerring (232) in ausreichendem Kontakt miteinander stehen, um die Werkzeugwalze (210) und die Ambosswalze (212) zueinander gegenläufig zu drehen,

   wobei D1 + D2 = D3 + D4 und
   wobei ein Drehzahlverhältnis SR gleich

$$SR = \frac{VA}{VS} = \frac{D3}{D1} \times \frac{D2}{D4}$$

   ist und im Bereich von über 1,02 bis 1,25 liegt.

2. Rotierende Vorrichtung (200) nach Anspruch 1, wobei das Trennwerkzeug 214 eine flexible Klinge oder eine Schneidform (201) ist, deren Trennkante so konstruiert ist, dass sie Trennkantenabschnitte einschließt, die ausgewählt sind aus einer Gruppe, die **dadurch gekennzeichnet ist, dass** sie einen geradlinigen Abschnitt, einen krummlinigen Abschnitt und jede Kombination davon umfasst.

3. Rotierende Vorrichtung (200) nach einem der vorangehenden Ansprüche, wobei das Trennwerkzeug (214) aus einem Material besteht, das ausgewählt ist aus der Gruppe, bestehend aus Werkzeugstahl, Carbiden, Keramiken, Zementen und jeder Kombination davon.

4. Rotierende Vorrichtung (200) nach einem der vorangehenden Ansprüche, wobei der Antrieb ausgewählt ist aus einer Gruppe, die aus einem Motor (224) und einer Übersetzung, die mit der Werkzeugwalze und der Ambosswalze kommunizieren, und zwei Motoren, einem ersten Motor, der mit der Werkzeugwalze kommuniziert, und einem zweiten Motor, der mit der Ambosswalze kommuniziert, besteht.

5. Rotierende Vorrichtung (200) nach einem der vorangehenden Ansprüche, wobei der Trägerring (230) an einem Lager angeordnet ist, das zur Werkzeugwalze (210) gehört.

6. Rotierende Vorrichtung (200) nach einem der vorangehenden Ansprüche, wobei der Trägerring (230) an einem Lager angeordnet ist, das zur Ambosswalze (212) gehört.

**Revendications**

1.  Appareil rotatif (200) approprié pour trancher un matériau de nappe (202), l'appareil **caractérisé en ce qu'**il comprend :

    (a) un rouleau outil (210) susceptible de tourner autour d'un axe de rouleau outil, le rouleau outil (210) **caractérisé en ce qu'**il comprend :

      (i) au moins un outil tranchant (214) ayant un bord tranchant susceptible de tourner à un diamètre D1 autour de l'axe du rouleau outil à une vitesse linéaire de bord tranchant VS ;
      (ii) au moins un anneau porteur d'outil susceptible de tourner autour de l'axe de rouleau outil à un diamètre D3 ;

    (b) un contre-rouleau (212) disposé sensiblement parallèlement et à l'opposé du rouleau outil 210 et susceptible de tourner autour d'un axe de contre-rouleau, le contre-rouleau **caractérisé en ce qu'**il comprend :

      (i) une surface d'enclume susceptible de tourner à un diamètre D2 autour de l'axe de contre-rouleau à une vitesse linéaire d'enclume VA ;
      (ii) au moins un anneau porteur d'enclume (232) disposé à l'opposé du au moins un anneau porteur d'outil (230) et susceptible de tourner autour de l'axe de contre-rouleau à un diamètre D4 et disposé à l'opposé du au moins un anneau porteur d'outil (230), le au moins un anneau porteur d'outil 230 et le au moins un anneau porteur de contre-rouleau (232) étant en contact l'un avec l'autre, approprié pour faire tourner en sens contraire le rouleau outil (210) et le contre-rouleau (212) en rapport l'un avec l'autre,

    dans lequel D1 + D2 = D3 + D4, et
    dans lequel un rapport de vitesse SR est égal

    $$SR = \frac{VA}{VS} = \frac{D3}{D1} \text{ x } \frac{D2}{D4}$$

    et allant de plus de 1,02 à 1,25.

2.  Appareil rotatif (200) selon la revendication 1, dans lequel l'outil tranchant 214 est une lame souple ou une matrice (201) ayant le bord tranchant configuré pour inclure des parties de bord tranchant choisies dans le groupe **caractérisé en ce qu'**il comprend une partie rectiligne, une partie curviligne, et n'importe quelle combinaison de celles-ci.

3.  Appareil rotatif (200) selon l'une quelconque des revendications précédentes, dans lequel l'outil tranchant (214) est fabriqué à partir d'un matériau choisi dans le groupe constitué d'acier à outil, carbures, céramiques, cermets, et n'importe laquelle de leurs combinaisons.

4.  Appareil rotatif (200) selon l'une quelconque des revendications précédentes, dans lequel l'entraînement est choisi dans le groupe constitué d'un moteur (224) et d'un rapport d'engrenage communiquant avec le rouleau outil et le contre-rouleau, et deux moteurs, un premier moteur communiquant avec le rouleau outil et un deuxième moteur communiquant avec le contre-rouleau.

5.  Appareil rotatif (200) selon l'une quelconque des revendications précédentes, dans lequel l'anneau porteur (230) est disposé sur un support lié au rouleau outil (210).

6.  Appareil rotatif (200) selon l'une quelconque des revendications précédentes, dans lequel l'anneau porteur (230) est disposé sur un support lié au contre-rouleau (212).

Fig. 1

Fig. 2

Fig. 4

Fig. 3

Fig. 5    Fig. 6    Fig. 7

Fig. 8

Fig. 9

Fig. 10

Fig. 12

Fig. 11

Fig. 13

Fig. 14

EP 1 531 975 B1